# EUROPEAN PATENT SPECIFICATION

(11) **EP 2 921 562 B1**
(45) Date of publication and mention of the grant of the patent: **08.03.2017**
(21) Application number: 15163160.3
(22) Date of filing: 27.05.2011
(51) Int. Cl.: C12Q 1/68, C12N 15/09, G01N 33/53

(54) **METHOD OF DETECTING OBESITY RISK**
VERFAHREN ZUM NACHWEIS VON ADIPOSITASRISIKO
PROCÉDÉ DE DÉTECTION D'UN RISQUE D'OBÉSITÉ

(30) Priority: 27.05.2010 JP 2010122148
(43) Date of publication of application: 23.09.2015
(62) Divisional of application: 11786754.9
(73) Proprietor: The University of Tokyo, Bunkyo-ku, Tokyo 113-8654 (JP); Sekisui Medical Co., Ltd., Tokyo 103-0027 (JP)
(72) Inventor: Kadowaki, Takashi, Tokyo 113-8654 (JP); Ueki, Kohjiro, Tokyo 113-8654 (JP); Okazaki, Yukiko, Tokyo 113-8654 (JP); Bluher, Matthias, 04003 Leipzig (DE); Ozawa, Sumiko, Tokyo 103-0027 (JP)
(74) Representative: Hoffmann Eitle

(56) References cited:
- WO-A2-2008/109779
- A. MUKHERJEE ET AL: "FSTL3 deletion reveals roles for TGF-beta family ligands in glucose and fat homeostasis in adults", PROCEEDINGS OF THE NATIONAL ACADEMY OF SCIENCES, vol. 104, no. 4, 23 January 2007 (2007-01-23), pages 1348-1353, XP055081057, ISSN: 0027-8424, DOI: 10.1073/pnas.0607966104
- D. L. ALLEN ET AL: "Myostatin, activin receptor IIb, and follistatin-like-3 gene expression are altered in adipose tissue and skeletal muscle of obese mice", AJP: ENDOCRINOLOGY AND METABOLISM, vol. 294, no. 5, 19 February 2008 (2008-02-19), pages E918-E927, XP055081060, ISSN: 0193-1849, DOI: 10.1152/ajpendo.00798.2007
- R. THADHANI ET AL: "First-Trimester Follistatin-Like-3 Levels in Pregnancies Complicated by Subsequent Gestational Diabetes Mellitus", DIABETES CARE, vol. 33, no. 3, 1 March 2010 (2010-03-01), pages 664-669, XP055081063, ISSN: 0149-5992, DOI: 10.2337/dc09-1745
- PEIRIS HASSENDRINI N ET AL: "Brief Communication: Sexual dimorphic expression of myostatin and follistatin like-3 in a rat trans-generational under-nutrition model", NUTRITION & METABOLISM, BIOMED CENTRAL. LONDON, GB, vol. 7, no. 1, 20 May 2010 (2010-05-20), page 44, XP021080313, ISSN: 1743-7075, DOI: 10.1186/1743-7075-7-44

## Description

### TECHNICAL FIELD

The present invention relates to a method of detecting obesity risk in an individual as defined in claim 1.

### BACKGROUND ART

The root cause of type 2 diabetes mellitus is obesity due to high-fat diet and lack of exercise and an increase in insulin resistance associated therewith. Obesity (particularly, visceral fat obesity) and insulin resistance are a primary mechanism of risk accumulation in the metabolic syndrome. Therefore, it is extremely important to clarify the cause of obesity and insulin resistance and establish a prevention method and a treatment method based thereon for prevention/treatment of diseases such as arteriosclerosis and cardiovascular disease having type 2 diabetes mellitus as a background factor; however, the cause is not yet completely clarified.

Researches are recently made on the relevance of size distribution of adipocytes in human fat tissue, their morphological changes, and expression of various genes in those cells, as well as the regulation of glucose and lipid metabolism at individual level. For example, in Patent Literature 1, the gene expression of adipocytes of type-2 diabetic patients is analyzed to search for up-regulated and down-regulated genes. However, Patent Literature 1 only describes the extraction of a group of genes which varied in the level of expression between type-2 diabetic patients and healthy individuals through global analysis, does not describe the relevance of the extracted genes for actual diseases (e.g., clinical conditions such as obesity and insulin resistance), and does not clarify how the extracted genes behave as gene markers related to type 2 diabetes mellitus and what type of diagnosis the extracted genes can be used for.

In Patent Literature 2, gene expression in liver tissue of type-2 diabetic patients is analyzed to search for enhanced (up-regulated) and attenuated (down-regulated) genes. Analysis is also performed on correlation between the searched genes and indexes (BMI, serum HbA1c, HOMA-R, MCR) related to the clinical conditions of type 2 diabetes mellitus, and the relevance of the extracted genes for actual diseases (e.g., clinical conditions such as obesity and insulin resistance) is studied. However, the individuals studied are type-2 diabetic patients and it is uncertain whether variations in extracted gene expression are the cause or the result of the clinical conditions of type 2 diabetes mellitus.

Follistatin-like 3 (FSTL3) is a secretory glycoprotein binding to activin and myostatin (Non-Patent Literatures 1 and 2). This protein consists of a signal peptide and two tandem segments including a follistatin-like domain (SMART accession SM00274) and a Kazal-like serine protease inhibitor domain (SMART accession SM00280). FSTL3 is mainly expressed in placenta, ovary, uterus, and testis and is also expressed in tissues of skin, heart, lung, and kidney.

Activin is a cytokine belonging to the TGF-β super family and has a dimer structure as is the case with other TGF-βs. Due to differences in constituent subunits (called β-subunits), three major activin forms, activin A (βA-βA), activin AB (βA-βB), and activin B (βB-βB), which are dimer polypeptide-growth factors, are known. An activin B receptor, ALK7, is reported as a negative regulator of the pancreatic β-cell function (Non-Patent Literature 3).

With regard to the expression of FSTL3 in adipocytes, Patent Literature 1, for example, reports that the expression is down-regulated in diabetic patients as compared to healthy individuals. However, correlations between the FSTL3 expression and the obesity risk, the diabetes onset risk, or various clinical conditions in diabetic patients (such as insulin resistance and obesity) are not described, and thus, the effectiveness of FSTL3 as a diagnostic marker is not clear.

The study of Mukherjee, A et al. describes that homozygous FSTL3 knockout adult mice develope a distinct group of metabolic phenotypes. Furthermore, the results demonstrate that FSTL3 regulation of activin and myostatin is critical for normal adult metabolic homeostasis ("FSTL3 deletion reveals roles for TGF-β family ligands in glucose and fat homeostasis in adults." Proceedings of the National Academy of Sciences 104.4 (2007): 1348-1353).

Allen, David L., et al. sought to determine whether expression of MSTN, its receptor activin RIIb (ActRIIb), or its binding protein follistatin-like-3 (FSTL3) are altered in subcutaneous or visceral adipose or in skeletal muscle in response to obesity. The results demonstrate that expression of MSTN and its associated binding proteins can be modulated in adipose tissue and skeletal muscle by chronic obesity ("Myostatin, activin receptor IIb, and follistatin-like-3 gene expression are altered in adipose tissue and skeletal muscle of obese mice." American Journal of Physiology-Endocninology and Metabolism 294.5 (2008): E918-E927).

Thadhani, Ravi, et al. measure FSTL3 levels in serum collected during the first trimester of pregnancy. Logistic regression analysis was used to determine the risk of gestational diabetes mellitus (GDM). The first-trimester FSTL3 levels are associated with glucose intolerance and GDM later in pregnancy ("First-trimester follistatin-like-3 levels in pregnancies complicated by subsequent gestational diabetes mellitus." Diabetes Care 33.3 (2010): 664-669).

Peiris, Hassendrini N., et al. followed the hypothesis of an involvement of myostatin in glucose metabolism and adipogenesis. The sexual dimorphism was clearly evident in the majority of the measures, including myostatin and FSTL-3 expression. In summary, myostatin may prove to be a key mediator of the effects of inadequate prenatal nutrition, independently or in combination with a high-fat postnatal diet on offspring phenotype ("Brief Communication: Sexual dimorphic expression of myostatin and follistatin like-3 in a rat trans-generational under-nutrition model." Nutrition & metabolism 7.1 (2010): 1.).

### CITATION LIST

### PATENT LITERATURE

Patent Literature 1: Japanese Laid-Open Patent Publication No. 2005-114725
Patent Literature 2: Japanese Laid-Open Patent Publication No. 2006-296346

### NON PATENT LITERATURE

Non-Patent Literature 1: J. Biol. Chem. 275: 40788-96 (2000)
Non-Patent Literature 2: J. Biol. Chem. 277: 40735-41 (2002)
Non-Patent Literature 3: Proc. Natl. Acad. Sci. U. S. A. 2008 May 20; 105(20): 7246-51

### SUMMARY OF INVENTION

The present invention is defined in the claims.

### Preferred embodiments

Item 1. A method of detecting a risk of developing obesity, comprising
   (i) a step of measuring an expression level of FSTL3 gene in a biological sample selected from the group consisting of blood sample, serum sample, plasma sample, visceral fat tissue sample and subcutaneous fat tissue sample derived from an individual having a BMI value less than 25, wherein
      if the expression level of FSTL3 gene in the biological sample is increased by a certain multiple or more as compared to a standard value S_{FSTL3}, it is determined that the risk of developing obesity is high.
Item 2. The method according to claim 1, further comprising
   (ii) a step of measuring an expression level of inhibin βB gene in the biological sample, wherein
      if the expression level of FSTL3 gene in the biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}, it is determined that the risk of developing obesity is high.
Also disclosed is a method of measuring the level of FSTL3 gene expression in a biological sample and correlating the measured level of expression with the detection of a risk of developing diabetes. The present disclosure also provides a method of measuring the level of FSTL3 gene expression in an individual with a BMI value less than 25 not clinically determined as obesity and correlating the measured level of expression with the detection of a risk of developing obesity. The present disclosure further provides a method of measuring the level of FSTL3 gene expression in an individual with a BMI value less than 25 and correlating the measured level of expression with the detection of a risk of developing diabetes.

The present disclosure further provides a method of measuring the level of inhibin βB gene expression and correlating the ratio of expression level of FSTL3 gene to inhibin βB gene with the detection of a risk of developing obesity or diabetes.

The inventors have performed a global analysis of genes having expression levels varied in association with obesity and insulin resistance in adipocytes and discovered that the mRNA expression level of FSTL3 in Japanese subjects with a BMI value of 22 or higher not diagnosed as having type 2 diabetes mellitus is increased as compared to subjects with a BMI value less than 22. The inventors also discovered that German subjects have positive correlation between the mRNA expression level of FSTL3 and obesity indices such as BMI value, thereby completing the present invention. Said FSTL3 is reported as a factor down-regulated (reduced in production) in type 2 diabetes mellitus in Patent Literature 1.

Therefore, the present disclosure relates to the followings.
(1) A method of correlating the expression level of FSTL3 gene in a biological sample with the detection of a degree of risk of developing diabetes, comprising:
   (i) a step of measuring the expression level of FSTL3 gene in a biological sample, wherein
      if the expression level of FSTL3 gene in the biological sample is increased by a certain multiple or more as compared to a standard value S_{FSTL3}, it is determined that the risk of developing diabetes is high.
(2) A method of correlating the expression level of FSTL3 gene in a biological sample derived from an individual having a BMI value less than 25 with the detection of a degree of risk of developing diabetes, comprising:
   (i) a step of measuring the expression level of FSTL3 gene in a biological sample derived from an individual having a BMI value less than 25, wherein
      if the expression level of FSTL3 gene in the biological sample is increased by a certain multiple or more as compared to a standard value S_{FSTL3}, it is determined that the risk of developing diabetes is high.
(3) A method of correlating the expression level of FSTL3 gene in a biological sample derived from an individual having a BMI value less than 25 with the detection of a degree of risk of developing obesity, comprising:
   (i) a step of measuring the expression level of FSTL3 gene in a biological sample derived from an individual having a BMI value less than 25, wherein
      if the expression level of FSTL3 gene in the biological sample is increased by a certain multiple or more as compared to a standard value S_{FSTL3}, it is determined that the risk of developing obesity is high.
(4) A method of correlating the ratio of expression level of FSTL3 gene to inhibin βB gene in a biological sample with the detection of a degree of risk of developing diabetes, comprising:
   (i) a step of measuring the expression level of FSTL3 gene in a biological sample; and
   (ii) a step of measuring the expression level of inhibin βB gene in the biological sample, wherein
   if the expression level of FSTL3 gene in the biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}, it is determined that the risk of developing diabetes is high.
(5) A method of correlating the ratio of expression level of FSTL3 gene to inhibin βB gene in a biological sample derived from an individual having a BMI value less than 25 with the detection of a degree of risk of developing diabetes, comprising:
   (i) a step of measuring the expression level of FSTL3 gene in a biological sample derived from an individual having a BMI value less than 25; and
   (ii) a step of measuring the expression level of inhibin βB gene in the biological sample, wherein
      if the expression level of FSTL3 gene in the biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}, it is determined that the risk of developing diabetes is high.
(6) A method of correlating the ratio of expression level of FSTL3 gene to inhibin βB gene in a biological sample derived from an individual having a BMI value less than 25 with the detection of a degree of risk of developing obesity, comprising:
   (i) a step of measuring the expression level of FSTL3 gene in a biological sample derived from an individual having a BMI value less than 25; and
   (ii) a step of measuring the expression level of inhibin βB gene in the biological sample, wherein
      if the expression level of FSTL3 gene in the biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}, it is determined that the risk of developing obesity is high.
(7) A kit for correlating the expression level of FSTL3 gene in a biological sample with the detection of a degree of risk of developing diabetes, comprising:
   (i) a probe for measuring the expression level of FSTL3 gene in a biological sample; and
   (ii) an instruction teaching that the risk of developing diabetes is high if the expression level of FSTL3 gene in the biological sample is increased by a certain multiple or more as compared to a standard value S_{FSTL3}.
(8) A kit for correlating the expression level of FSTL3 gene in a biological sample derived from an individual having a BMI value less than 25 with the detection of a degree of risk of developing diabetes, comprising:
   (i) a probe for measuring the expression level of FSTL3 gene in a biological sample; and
   (ii) an instruction teaching that the risk of developing diabetes is high if the expression level of FSTL3 gene in a biological sample derived from an individual having a BMI value less than 25 is increased by a certain multiple or more as compared to a standard value S_{FSTL3}.
(9) A kit for correlating the expression level of FSTL3 gene in a biological sample derived from an individual having a BMI value less than 25 with the detection of a degree of risk of developing obesity, comprising:
   (i) a probe for measuring the expression level of FSTL3 gene in a biological sample; and
   (ii) an instruction teaching that the risk of developing obesity is high if the expression level of FSTL3 gene in a biological sample derived from an individual having a BMI value less than 25 is increased by a certain multiple or more as compared to a standard value S_{FSTL3}.
(10) A kit for correlating the ratio of expression level of FSTL3 gene to inhibin βB gene in a biological sample with the detection of a degree of risk of developing diabetes, comprising:
   (i) a probe for measuring the expression level of FSTL3 gene in a biological sample;
   (ii) a probe for measuring the expression level of inhibin βB gene in a biological sample; and
   (iii) an instruction teaching that the risk of developing diabetes is high if the expression level of FSTL3 gene in a biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}.
(11) A kit for correlating the ratio of expression level of FSTL3 gene to inhibin βB gene in a biological sample derived from an individual having a BMI value less than 25 with the detection of a degree of risk of developing diabetes, comprising:
   (i) a probe for measuring the expression level of FSTL3 gene in a biological sample;
   (ii) a probe for measuring the expression level of inhibin βB gene in a biological sample; and
   (iii) an instruction teaching that the risk of developing diabetes is high if the expression level of FSTL3 gene in a biological sample derived from an individual having a BMI value less than 25 is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}.
(12) A kit for correlating the ratio of expression level of FSTL3 gene to inhibin βB gene in a biological sample derived from an individual having a BMI value less than 25 with the detection of a degree of risk of developing obesity, comprising:
   (i) a probe for measuring the expression level of FSTL3 gene in a biological sample;
   (ii) a probe for measuring the expression level of inhibin βB gene in a biological sample; and
   (iii) an instruction teaching that the risk of developing obesity is high if the expression level of FSTL3 gene in a biological sample derived from an individual having a BMI value less than 25 is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}.

### ADVANTAGEOUS EFFECTS OF INVENTION

By using the present invention, a risk of developing obesity (hereinafter sometimes referred to as "obesity risk"). can be detected in early phase.

### BRIEF DESCRIPTION OF DRAWINGS

[Fig. 1] Fig. 1 depicts an experiment scheme for identifying a key molecule of the metabolic syndrome.
[Fig. 2] Fig. 2 depicts candidate genes of secretory proteins recognized as having correlation with obesity in cDNA microarray analysis.
[Fig. 3] Fig. 3 is a graph with the logarithmic values of FSTL3 mRNA expression levels plotted to BMI values for 188 cases of German subjects.
[Fig. 4] Fig. 4 is a graph with the logarithmic values of FSTL3 mRNA expression levels plotted to abdominal circumferences (cm) for 188 cases of German subjects.
[Fig. 5] Fig. 5 is a graph with the logarithmic values of FSTL3 mRNA expression levels plotted to external glucose infusion rates (GIR) in a glucose clamp test for 188 cases of German subjects.
[Fig. 6] Fig. 6 depicts a result of examination of tissue-specific expression of FSTL3 mRNA in control mice and *db*/*db* mice with Northern blotting method.
[Fig. 7] Fig. 7 depicts that expression of FSTL3 mRNA rises in mice that have become obese due to feeding of high-fat diet.
[Fig. 8] Fig. 8 is a diagram depicting that FSTL3 is not expressed in preadipocytes and is expressed in mature adipocytes.
[Fig. 9] Fig. 9 depicts the presence of positive correlation between expression of inhibin βB and BMI in early obesity, and also depicts the up-regulation of FSTL3 due to Actibin B.
[Fig. 10] Fig. 10 is a conceptual diagram depicting that FSTL3 binds to activin B and inhibits the binding of activin B to a receptor to suppress the antidiabetic effect of activin B.
[Fig. 11] Fig. 11 is a graph with the ratio of (FSTL3 mRNA expression level)/(inhibin βB mRNA expression level) plotted to BMI values for 188 cases of German subjects.
[Fig. 12] Fig. 12 is a graph comparing blood glucose levels between mice with FSTL3 gene expressed by adenovirus in the liver and control mice until 120 minutes had elapsed in a glucose load test using C57BL/6J mice.
[Fig. 13] Fig. 13 is a graph comparing blood glucose levels between *db*/*db* mice administered FSTL3 antisense oligonucleotides and control *db*/*db* mice until 120 minutes had elapsed in a glucose load test.
[Fig. 14] Fig. 14 is a graph of Akt phosphorylation in the liver and the skeletal muscle in *db*/*db* mice administered FSTL3 antisense oligonucleotides and control *db*/*db* mice.
[Fig. 15] Fig. 15 is a graph of correlation between insulin sensitivity and FSTL3 for 50 cases of German subjects with BMI less than 25.

### DESCRIPTION OF EMBODIMENTS

The present invention was achieved based on new findings that FSTL3, conventionally recognized as a factor down-regulated (reduced in production) in type 2 diabetes mellitus, has positive correlation with indices of obesity such as BMI value. The present invention will now be described in detail.

In this description, the following terms are defined as follows.

Healthy Individual: A "healthy individual" refers to an individual with a fasting blood glucose level less than 110 mg/dL or a two-hour post-load glucose level (2-hour PG) less than 140 mg/dL and a BMI value less than 25.

BMI: BMI (Body Mass Index) is an index evaluating balance between body height and body weight and is calculated from the formula of (body weight (kg))/(body height (m))². A BMI value equal to or greater than 25 is determined as obesity The term "BMI" as used herein is intended to refer to the index itself; however, "BMI" is used in the same meaning as a "BMI value" without distinction in some cases.

Subject: The term "subject" as used herein includes both human and animal.

Certain Multiple: The term "a certain multiple" as used herein means a real number greater than one.

### (Biological Sample)

The term "biological sample" as used herein means a biological sample selected from the group consisting of blood, serum, plasma, and visceral and subcutaneous fat tissues. Cells derived from fat tissues can be collected with any known methods such as ERCP, secretin stimulation, fine-needle aspiration, cytological brushing, and large-diameter needle biopsy.

### (Detection and/or Measurement of FSTL3 Gene and/or inhibin βB Gene Expression)

Detection and/or measurement of FSTL3 gene and/or inhibin βB gene may include, for example, detection of the increase, decrease, and/or absence of a certain nucleic acid molecule such as mRNA (or cDNA), measurement/detection of an expressed polypeptide/protein, and measurement/detection of bioactivity (or absence thereof) of an expressed protein/polypeptide. The bioactivity may include enzyme activity, for example, activity related to signal-transducing pathways such as antigen recognition and effector events.

### ("Detection" and "Measurement")

The term "detection" as used herein refers to qualitative or quantitative determination of the presence of mRNA and/or polypeptide. The term "measurement" as used herein refers to the quantitative determination of the expression level of mRNA and/or polypeptide in a biological sample.

### (Method of Detecting and/or Measuring mRNA in Biological Sample)

For a method of detecting and/or measuring mRNA in a biological sample, well-known methods in this technical field may be utilized. For example, gene amplification may be performed by using a nucleotide including all or a portion of the base sequence of FSTL3 gene as a probe or primer to measure the level of gene expression. The level of gene expression can be measured by methods such as Northern blotting method, the RT-PCR method, the real-time PCR method, the *in situ* hybridization method, and a method using a microarray (such as a gene chip), and all of these methods can be implemented by known methods. Gene expression may be measured as an amount of mRNA transcribed from a portion of the gene and can be measured by using a nucleotide probe or primer which hybridizes to the mRNA. A base length of the probe or primer is 10 to 50 nucleotides, preferably, 15 to 25 nucleotides.

### (Method of Detecting and/or Measuring Polypeptide in Biological Sample)

For a method of detecting and/or measuring a polypeptide in a biological sample, well-known methods in this technical field may be utilized. For example, the methods include, but not limited to, Western blotting method, the ELISA method or RIA method, the chemiluminescent immunoassay method (CLIA method), the latex turbidimetric immunoassay method (LTIA method), and the enzyme immunoassay method utilizing electrochemical luminescence.

### (Antibody)

For the purpose of detecting a polypeptide or a peptide fragment, a monoclonal antibody or a polyclonal antibody recognizing the polypeptide or a peptide fragment thereof can be produced by immunizing a mouse or a rabbit with purified protein, for example. A known antibody recognizing the polypeptide or a peptide fragment thereof is also usable. The preparation, usage, etc., of antibodies are described in Harlow, E. and Lane, D., Antibodies: A laboratory Manual, (1988), Cold Spring Harbor Laboratory Press.

An antibody such as a polyclonal antibody is usable for detecting a peptide of the present invention in Western blotting method. An example of a method of detecting a marker is the ELISA method using an antibody capable of capturing an antigen and a secondary antibody capable of detecting the captured antigen.

### (Comparison with Gene Expression Level of Healthy Individuals)

One aspect of the present invention includes a step of comparing the expression level of FSTL3 gene in a biological sample selected from the group consisting of blood sample, serum sample, plasma sample, visceral fat tissue sample and subcutaneous fat tissue sample derived from a subject with the expression level of FSTL3 gene in healthy individuals (standard value S_{FSTL3}). For the expression level of FSTL3 gene in healthy individuals, a value acquired by statistically processing the expression levels of FSTL3 gene in a plurality of healthy individuals is usable, for example. In the present invention, if the expression level is increased by a certain multiple or more as compared to the standard value, it can be determined that the risk of developing obesity is high.

For the standard value, S_{FSTL3}, of the expression level of FSTL3 gene, an average value of expression levels of FSTL3 gene in biological samples derived from individuals with a BMI value less than 22 is usable, for example.

With regard to the "certain multiple", if the expression level is increased by a factor of 1000, preferably, by a factor of 100, more preferably, by a factor of 5 to 10, more preferably, by a factor of 3, more preferably, by a factor of 2, more preferably, by a factor of 1.5, it can be determined that the risk of developing obesity is high.

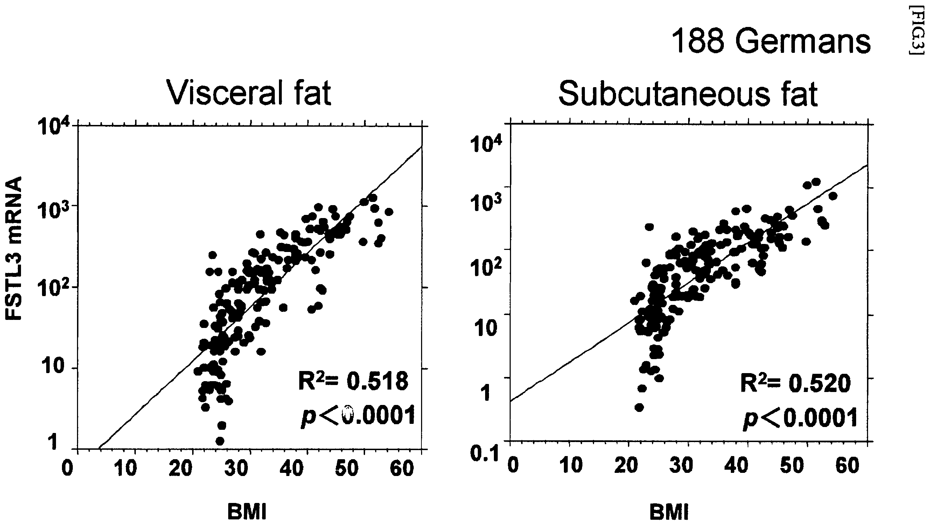

If favorable correlation exists between FSTL3 mRNA expression level and BMI value as depicted in Fig. 3 of the second example described later, the FSTL3 mRNA expression level acquired by substituting a BMI value of 18 in an equation of an approximate curve is about 10 in the case of visceral fat of Fig. 3 and, therefore, for example, if the FSTL3 mRNA expression level is 15 or more, it can be determined that the risk of developing obesity is high.

### (Comparison with Protein Expression Level of Healthy Individuals)

One aspect of the present invention includes a step of comparing an expression level of the FSTL3 protein in a biological sample selected from the group consisting of blood sample, serum sample, plasma [0024] sample, visceral fat tissue sample and subcutaneous fat tissue sample derived from a subject with an expression level (standard value S_{FSTL3}) in healthy individuals. For the expression level of the protein in healthy individuals, a value acquired by statistically processing the FSTL3 protein expression levels in a plurality of healthy individuals is usable, for example. In the present invention, if the expression level is increased by a certain multiple or more as compared to the standard value, it can be determined that the risk of developing obesity and diabetes is high.

### (Kit)

The present disclosure provides a kit for correlating the expression level of FSTL3 gene in a biological sample with the detection of a degree of risk of developing diabetes, or a kit for correlating the expression level of FSTL3 gene in a biological sample with the detection of a degree of risk of developing obesity, the kit including (i) and (ii) as follows:
(i) a probe for measuring the expression level of FSTL3 gene in a biological sample; and
(ii) an instruction teaching that the risk of developing diabetes is high or the risk of developing obesity is high if the expression level of FSTL3 gene in a biological sample is increased by a certain multiple or more as compared to the standard value S_{FSTL3} (the contents of the teaching correspond to the purpose of measurement).

The present disclosure also provides a kit for correlating the ratio of expression level of FSTL3 gene to inhibin βB gene in a biological sample with the detection of a degree of risk of developing diabetes, or a kit for correlating the said ratio with the detection of a degree of risk of developing obesity, the kit including (i) to (iii) as follows:
(i) a probe for measuring the expression level of FSTL3 gene in a biological sample;
(ii) a probe for measuring the expression level of inhibin βB gene in a biological sample; and
(iii) an instruction teaching that the risk of developing diabetes is high or the risk of developing obesity is high if the expression level of FSTL3 gene in a biological sample is increased by a certain multiple or more as compared to the standard value S_{FSTL3} and the ratio of expression level of FSTL3 gene to inhibin βB gene in the biological sample is equal to or greater than a certain multiple of a standard value S_{FSTL3/ActB} (the details of the teaching correspond to the purpose of measurement).

The biological sample mentioned in relation to the kit above may be a biological sample derived from an individual with a BMI value less than 25.

The probes for measuring the expression levels of FSTL3 gene and/or inhibin βB gene are not particularly limited as long as the probes have specificity as a probe normally used for measuring gene expression levels, and the sequence and the length of the bases preferred for the probes can be selected in consideration of the target base sequence by a technique using a calculation formula or an empirical technique that is a method well-known to those skilled in the art. A preferred probe base length is 10 to 50 nucleotides, preferably, 15 to 25 nucleotides as described in the section of "(Method of Detecting and/or Measuring mRNA in Biological Sample)".

The instruction teaching a higher risk of developing diabetes or a higher risk of developing obesity may have descriptions of the purpose of measurement, a method of measurement, a guide for determination from the measurement result, etc., along with the teaching, and may have any name such as accompanying document and direction for use.

The present disclosure provides a kit capable of detecting the risk of developing obesity and/or diabetes including one or a plurality of anti-FSTL3 monoclonal antibodies or antigen-binding fragments thereof. Another kit provided by the present disclosure is a kit capable of detecting the risk of developing obesity and diabetes including one or a plurality of nucleic acids capable of specifically binding to a nucleic acid coding for FSTL3 gene.

The present disclosure provides a kit capable of detecting the risk of developing obesity and/or diabetes including one or a plurality of anti-activin B monoclonal antibodies or antigen-binding fragments thereof. Another kit provided by the present disclosure is a kit capable of detecting the risk of developing obesity and diabetes including one or a plurality of nucleic acids capable of specifically binding to a nucleic acid coding for activin B.

### EXAMPLES

### [Experimental Materials and Methods]

### <Human Fat Tissue>

At the time of cosmetic flap surgery, liposuction, stomach banding, gallstone removal surgery, appendectomy, or stomach cancer operation, visceral fat or subcutaneous fat was removed by the operator with the approval of subjects, frozen by liquid nitrogen, and stored at -80 °C.

### <mRNA Extraction>

For extraction of mRNA from the fat tissue, RNeasy (registered trademark) Lipid Tissue Midi Kit (manufactured by Qiagen) was used and operated in accordance with the recommended protocol. Details are as follows.

Up to 500 mg of human fat tissue was put into 5 ml of QIazol (registered trademark) Lysis Reagent and homogenized until a homogeneous sample was acquired. After adding 1 ml of chloroform, the sample was intensely stirred and centrifuged at 5,000×g for 15 minutes at 4 °C. An upper layer was removed and mixed with 3 mL of 70 % ethanol, transferred to a column set in a collection tube, and centrifuged at 5,000×g for 5 minutes at room temperature. The column was washed in two steps and RNA remaining in the column was extracted with RNase-free water as total RNA.

### <cDNA Microarray Analysis>

For gene chip measurement, a gene chip and a measurement device of Affymetrix, Inc. were used. The gene expression analysis with the gene chip was performed in conformity to the protocol recommended by Affymetrix. The procedure is as follows.

### (1) Probe Synthesis

### (i) Double-Stranded cDNA Synthesis

Double-stranded cDNA was synthesized from the prepared total RNA by using SUPERSCRIPTChoice System manufactured by Gibco BRL. Fifteen µg of total RNA and 100 pmol of T7-(dT)24 primer were dissolved into DEPC-treated water to make 11 µL of solution. After reaction for 10 minutes at 70 °C, the solution was ice-cooled and, 4 µL of 5×1st strand cDNA buffer (manufactured by Gibco BRL), 2 µL of 0.1×DTT (dithiothreitol manufactured by Gibco BRL), and 1 µL of 10 mM dNTP mix (manufactured by Gibco BRL) were added before keeping the temperature at 42 °C for 2 minutes. Two µg of reverse transcriptase (Superscript II RT manufactured by Invitrogen) was added and then reacted at 42 °C for 1 hour.

DEPC-treated water, 30 µL of 5 x2nd strand reaction buffer, 3 µL of 10 mM dNTP, 1 µL of 10 U/µL DNA ligase, 4 µL of 10 U/µL DNA polymerase I, and 2 µL of 2 U/µL RNaseH were added to and mixed with the reaction solution and then reacted at 16 °C for two hours. Subsequently, 2 µL of 5 U/µL T4 DNA polymerase was added and reacted at 16 °C for 5 minutes. To this solution, 10 µL of 0.5 M EDTA was added. Phenol-chloroform 1:1 mixed solution was added to this reaction solution and a tube containing these materials was vertically shaken for mixing purpose. The mixed solution was centrifuged at 15,000 rpm for 10 minutes at 4 °C and the aqueous layer was transferred to a new centrifugal tube. To this aqueous layer, 1/10th volume of 3 M sodium acetate and three times volume of 100 % ethanol were added and sufficiently mixed. The mixture was left for 10 minutes at -80 °C and then centrifuged at 15,000 rpm for 10 minutes at 4 °C. After precipitated pellets were rinsed twice with 70 % ethanol and dried for 5 minutes at room temperature, 12 µL of DEPC-treated water was added.

### (ii) Synthesis of Biotin-Labeled cRNA Probes

Biotin-labeled cRNA robes were synthesized from the double-stranded cDNA synthesized as above by using BioArray High Yield RNA Transcript Labeling Kit manufactured by Enzo Life Sciences, Inc. Five µL of the double-stranded cDNA, 17 µL of DEPC-treated water, 4 µL of 10×HY buffer, 4 µL of 10×biotin-labeled ribonucleotides, 4 µL of 10×DTT, 4 µL of 10×RNase inhibitor mix, and 2 µL of 20×T7 RNA polymerase were mixed and reacted for 4 hours at 37 °C.

Unreacted biotin-labeled ribonucleotides were removed from the biotin-labeled cRNA probe solution synthesized as above by using RNasey manufactured by Qiagen. To the biotin-labeled cRNA probe solution, 160 µL of DEPC-treated water was added; 700 µL of RLT buffer was mixed; and 500 µL of 100 % ethanol was added and sufficiently mixed. This solution was added by 700 µL each to RNeasy mini spin columns and centrifuged at 8,000 rpm for 15 seconds. The eluate was added again to the RNeasy mini spin column and centrifuged at 8,000 rpm for 15 seconds. Subsequently, 500 µL of RPE buffer was added to the RNeasy mini spin column and centrifuged at 8,000 rpm for 15 seconds. Five-hundred µL of RPE buffer was added again to the RNeasy mini spin column and centrifuged at 15,000 rpm for two minutes.

The RNeasy mini spin column having the biotin-labeled cRNA probes adsorbed and washed as described above was transferred to a new centrifugal tube. After adding 30µL of DEPC-treated water, the RNeasy mini spin column was left for one minute at room temperature. The column was centrifuged at 8,000 rpm for 15 seconds to elute a purified biotin-labeled cRNA probe solution.

The fragmentation of the purified biotin-labeled cRNA probe solution was then performed. The biotin-labeled cRNA probe solution and 5×Fragmentation buffer (1/5th volume of final solution volume was added) were mixed and adjusted to a biotin-labeled cRNA probe concentration of 0.5 µg/µL and reacted for 35 minutes at 94 °C. The fragmentation of the probes to a length of about 100 bases was confirmed by performing 1 % agarose gel electrophoresis.

### (2) Hybridization

In hybridization, the resultant fragmented biotin-labeled cRNA probes were evaluated by a test chip to confirm that no problem existed before performing a main test In the main test, a Human Genome U133 Plus 2.0 Array (hereinafter, human chip) was used. The hybridization was performed with the following procedure for both the test chip and the human chip.

Sixty µg of the fragmented biotin-labeled cRNA probes, 12 µL of control oligonucleotide B2 (5 nM), 12 µL of 100×control cRNA cocktail, 12 µL of herring sperm DNA (10 mg/mL), 12 µL of acetylated BSA (50 mg/mg), and 600 µL of 2×MES hybridization buffer were added and adjusted with DEPC-treated water to 1200 µL (hereinafter referred to as "hybridization cocktail"). The hybridization cocktail was heated for 5 minutes at 99 °C for heat denaturation and left for 5 minutes at 45 °C before centrifuging at 15,000 rpm for 5 minutes at room temperature. Eighty µL and 200 µL of supernatant were dispensed to the test chip and the human chip, respectively, and used for the hybridization.

The gene chips were returned to room temperature and prehybridized with 1×MES buffer (80 µL and 200 µL for the test chip and the human chip, respectively) at 45 °C for 10 minutes at 60 rpm. The prehybridization solution was removed and the heat-denatured hybridization cocktail was added for hybridization at 45 °C for 16 hours at 60 rpm.

### (3) Washing, Staining, and Scanning

The hybridization cocktail was removed from the chips and non-stringent wash buffer was added for washing and staining with a Fluidic station (manufactured by Affymetrix). After the washing and staining, the chips were scanned by a scanner to capture image data.

### (4) Data Analysis

The data analysis of the hybridization was performed by using an analysis software for Affymetrix, GeneSpring (Agilent Technologies Inc.). A global expression profile of reliable 24293 probes except those with a low expression level and miss-hybridization was studied.

### <Glucose Clamp Test>

A glucose clamp test was performed based on a method described in Am. J. Physiol. 1979 Sep; 237(3); E214-23. An infusion rate of insulin was set to 20 mIU/kg/min and a glucose injection rate was defined as an injection rate of 30 minutes of the latter half of the test.

### <Cell Lysis>

Cells were lysed by using Lysis Buffer containing 25 mM Tris-HCl of pH7.4,25 mM NaCl, 1 mM Na₃VO₄, 10 mM NaF, 10 mM Na₄P₂O₇, 1 mM EGTA, 10 nM okadaic acid, 5 mg/mL leupeptin, 5 mg/mL aprotinin and 1mM phenylmethylsulfonyl fluoride (PMSF), and 1 % Nonident-P 40, and centrifuged at 12,000 rpm for 20 minutes after ultrasonic treatment to use supernatant thereof.

### <Western Blot Analysis>

After electrophoresis of protein extracted from cells, gel was transferred to a PVDF (poly vinylidene difluoride) membrane. FLRG (N-18) (manufactured by Santa Cruz) and goat anti-rabbit IgG-HRP (manufactured by Santa Cruz) were used as a primary antibody and a secondary antibody, respectively, and after washing with buffer, BM Chemiluminescence Blotting Substrate (POD) (manufactured by Roche) was used for detection.

A semi-dry method was employed for blotting of protein and a PVDF membrane was used for a transfer membrane. For the detection of RGSH6-tagged protein, mouse anti-RGSH4 (manufactured by QIAGEN) and goat anti-mouse IgG (manufactured by BIO-RAD) were used as a primary antibody and a secondary antibody, respectively, and a chemiluminescence method was used for visualization. As a molecular-weight marker for Western blotting, 6×His Protein Ladder was used (molecular weight of 100, 75, 50,30, and 15 kDa, manufactured by QIAGEN).

### <Northern Blot Analysis>

Homogenization was performed in 1 mL of TRIzol (registered trademark) Reagent (manufactured by Invitrogen) per 50 to 100 mg of adipocytes. After adding 0.2 mL of chloroform, the homogenate was centrifuged at 12,000×g for 15 minutes at 4 °C and an upper aqueous layer was collected. After adding isopropanol, the aqueous layer was centrifuged at 12,000×g for 10 minutes at 4 °C to remove the liquid layer. Precipitated RNA was washed with 75 % ethanol, dried, and dissolved into RNase-free water.

The extracted RNA was electrophoresed in a denaturing gel and then transferred to a nylon membrane (Hybond-N⁺, manufactured by GE Healthcare Japan). Hybridization was performed by using [α-³²P]dCTP-labeled probes with a random primer method using Takara 6045 Random Primer DNA Labeling Kit. The nylon membrane after hybridization was imaged.

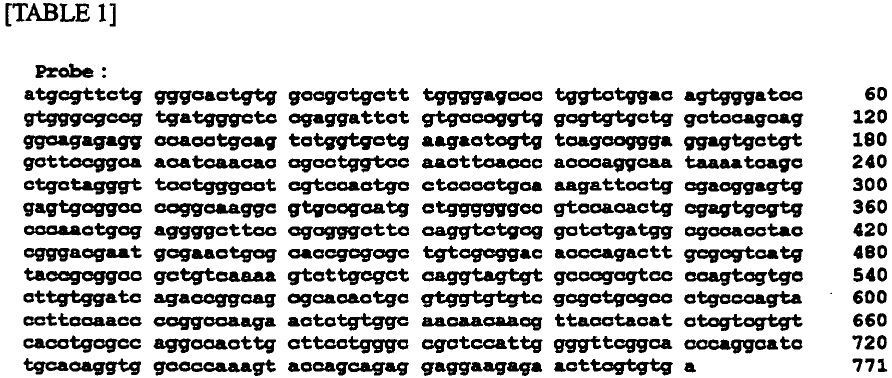

### <Production of Recombinant Adenovirus and Administration to Mouse>

The full length cDNA of mouse FSTL3 was introduced into pcDNA3.1/VS-HisA (manufactured by Invitrogen), excised by HindIII and EcoRV, and inserted according to the protocol of Takara Adenovirus Expression Vector Kit (manufactured by Takara) to produce adenovirus. The adenovirus was transfected to HEK293 cells by using CellPhect (registered trademark) Transfection Kit (manufactured by GE Healthcare) with the calcium phosphate method. The HEK293 cells after transfection were seeded on a 96-well plate to select those recognized as dead cells, and then, HEK293 cells on greater plates were infected to produce a larger and larger amount of adenovirus. After seven-week-old male B6 mice were adapted for one week, 5.0×10¹⁰ pfu of the adenovirus was dissolved in 150 µL of PBS and administered from the tail vein (eighth week, ninth week). After one week, a glucose load test was conducted.

### <Administration of Antisense Oligonucleotide>

After seven-week-old male B6 mice were adapted for one week, antisense oligonucleotide was intraperitoneally administered twice a week, a total of eight times (80 mg/body) and a glucose load test was conducted in the next week of the last administration (after 28 days from the first day of administration). The oligonucleotides used were as follows.

### [Chem 1]

• Sequence/Modification (= OligoA)
   5'-mG*mG*mG*mU*mU*G*G*A*A*G*G*T*A*C*T*G*G*G*C*mA*mG*mG*mG*mC-3' • control
   5'-mC*mG*mG*mG*mA*C*G*G*G*T*C*A*T*G*G*A*A*G*G*mU*mU*mG*mG*mG-3'
   (where * = Phosphorothioate Bonds, mN=2'-O-Methyl RNA base(2'-O-Me)

### < Measurement of Akt Phosphorylation >

Two-hundred mg each of the liver and the skeletal muscle were homogenized in Liver Buffer (having composition described in Table 2) containing 1 % Nonident-P 40. For the liver and the skeletal muscle, 7 mL and 4 mL of the Liver Buffer were respectively used. After centrifugation at 3,000 rpm for 10 minutes at 4 °C, supernatant was dispensed into a tube. The dispensed supernatant was ultracentrifuged at 55,000 rpm for 1 hour at 4 °C and supernatant was pipetted and dispensed into a tube. After protein quantification, SDS PAGE was performed with 10 % gel. After 30 minutes of blocking, reaction with a primary antibody (Phospho-Akt (Ser473): cell signaling #92715) diluted by a factor of 2000 was performed overnight at room temperature. Reaction with a secondary antibody (goat anti-rabbit IgG HRP (Santa Cruz sc-2054)) diluted by a factor of 5000 was performed for 2 hours at room temperature.

**[TABLE 2]**

| (Composition of Liver Buffer) | | |
|---|---|---|
| 10M Tris (pH 7.4) | 12.5 | mL |
| 0.5M NaF | 100 | mL |
| 100mM Na₄P₂0₇ (PPi) | 250 | mL |
| 0.2M EGTA | 25 | mL |
| 0.5M EDTA | 10 | mL |
| 200mM PMSF | 5 | mL Phenylmetylsulfonyl Fluoride |
| Complete | 10 | tabs (Roche 11 697 498 001) |
| Na₃VO₄ | 919.6 | mg (Sigma S6508, f. c.10 mM) |
| | 500 | mL |

After all the reagents were mixed, pH was adjusted to 7.4. Nonident-P 40 was added before used in experiments.

### [EXAMPLE 1]

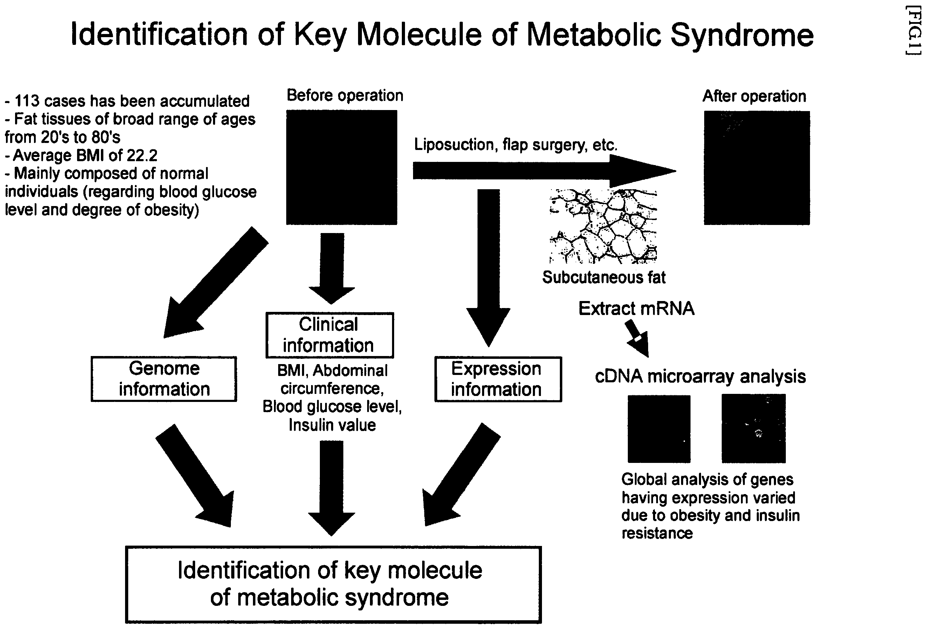

To identify key molecules of the metabolic syndrome, genes having expression varied due to obesity and insulin resistance were globally analyzed for the fat tissues of 113 cases of Japanese subjects. These cases were made up of abroad range of ages from twenties to eighties. The average BMI value of the subjects was 22.2. Although a BMI value equal to or greater than 25 is normally considered as obesity, this analysis was performed by classifying the cases with a BMI value equal to or greater than 22 as an overweight group (OW) and the cases with a BMI value less than 22 as a normal group (NW) (a BMI value of 22 is normally considered as standard body weight) and by integratively analyzing clinical information and expression information to attempt the identification of key molecules of the metabolic syndrome (see Fig. 1).

The clinical information of the subjects of this study is described in Table 3.

**[TABLE 3]**

| Clinical Information of Subjects | | |
|---|---|---|
| Clinical information | Male (n=18) | Female (n=95) |
| Age (year) | 51.4 ± 4.25 | 53.2 ± 1.61 |
| BMI | 25.0 ± 1.14 | 21.5 ± 0.29 |
| Systolic blood pressure (mmHg) | 118.4 ± 4.04 | 109.5 ± 1.58 |
| Diastolic blood pressure (mmHg) | 75.7 ± 3.27 | 68.8 ± 1.19 |
| Waist circumference size (cm) | 89.11 ± 3.32 | 72.5 ± 11.2 |
| Fasting blood glucose level (mg/dl) | 101.5 ± 7.79 | 91.1 ± 1.87 |
| HOMA-IR | 1.46 ± 0.34 | 1.28 ± 0.22 |
| HbA1c (%) | 4.98 ± 0.08 | 4.93 ± 0.04 |
| Total cholesterol (mg/dl) | 188.7 ± 9.65 | 199.6 ± 4.22 |
| Total cholesterol (mg/dl) | 499.6 ± 3.86 | 68.4 ± 1.76 |
| Neutral fat (mg/dl) | 116.1 ± 19.1 | 86.6 ± 5.37 |

Lower possibility of presence of secondary changes due to deterioration of the metabolic state is likely to enable the identification of genes such as those specifically appearing in early phase of obesity.

Among all the 113 cases, 18 cases were male and 95 cases were female. The table describes numerical numbers or values of age, BMI, systolic arterial pressure (mmHg), diastolic blood pressure (mmHg), waist circumference size (cm), fasting blood glucose level (mg/dL), HOMA-IR, HbAlc (%), total cholesterol (mg/dL), HDL cholesterol (mg/dL), and neutral fat (mg/dL). The numbers in the table represent average values ± standard errors.

Although the criterion for determining obesity based on BMI value is not absolute, Japanese Society for the Study of Obesity defines that a BMI value of 22 corresponds to a standard weight, that a BMI value of 25 or greater corresponds to obesity, and that a BMI value less than 18.5 corresponds to low body weight. As is clear from the numeric values of Table 3, although cases within a range considered as obesity in terms ofBMI value are included, the subjects of this study are mainly composed of normal individuals (in terms of blood glucose level and obesity degree). For example, an HOMA-IR value is an index of insulin resistance and it is determined under certain criteria that insulin resistance exists if an HOMA-IR value is greater than 2; however, the average value of the subjects of this study is 1.46, and diabetes has never developed in most of the cases.

The inventors conceived that these cases might have lower possibility of presence of secondary changes due to deterioration of the metabolic state, which is likely to enable the identification of genes such as those specifically appearing in early phase of obesity.

For each of the cases, mRNA was extracted from collected subcutaneous fat to perform the cDNA microarray analysis.

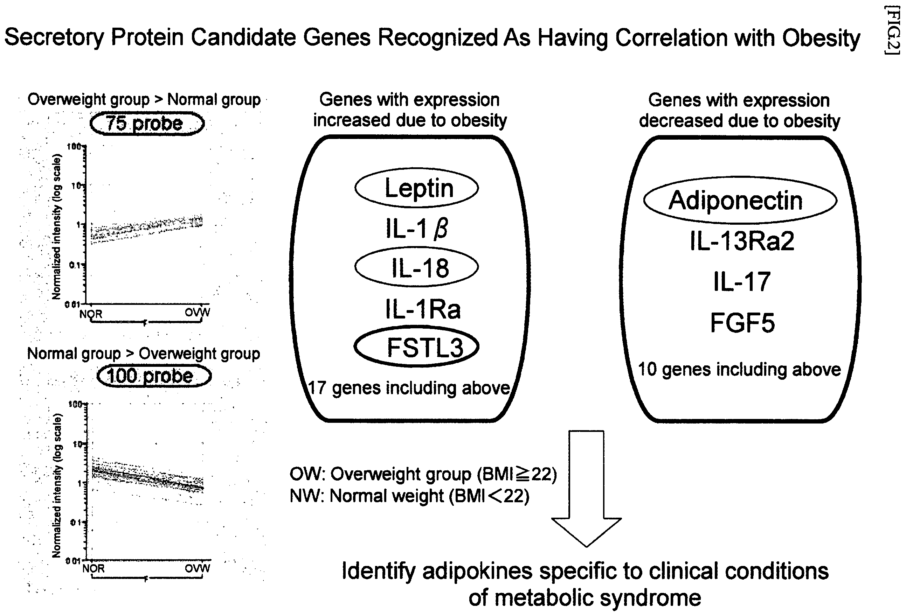

As a result, genes increased in expression due to obesity were detected, including leptin and IL-18 which have been previously-reported. Adiponectin was detected as a gene decreased in expression due to obesity, as was previously reported. Expected behaviors displayed by these known genes indicated that the experimental system of the cDNA microarray of this study worked well (see Fig. 2).

Follistatin-like 3 (FSTL3) reported as a "gene down-regulated in diabetes" in Patent Literature 1 (paragraphs 0058 and 0059) was surprisingly identified as a gene increased in expression due to obesity in this study. Superficially, this result seems contradictory.

### [EXAMPLE 2]

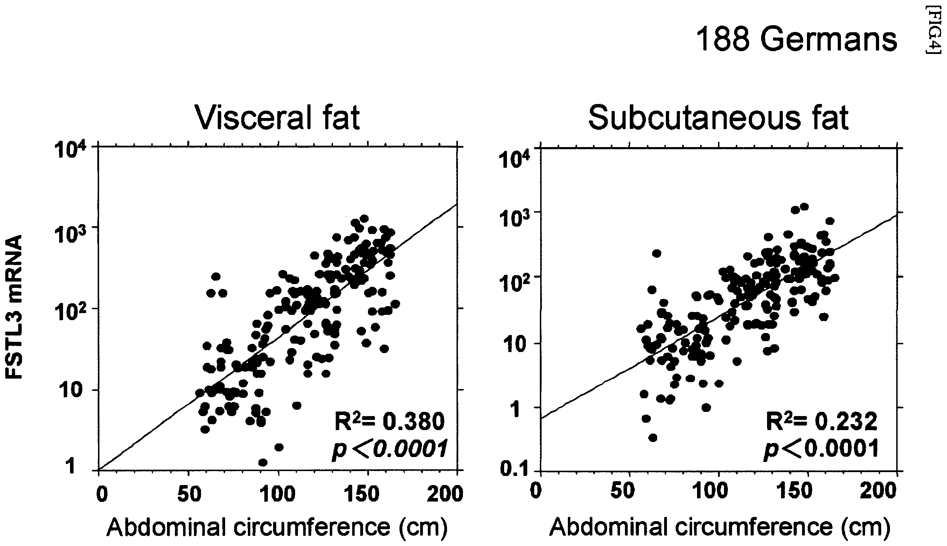

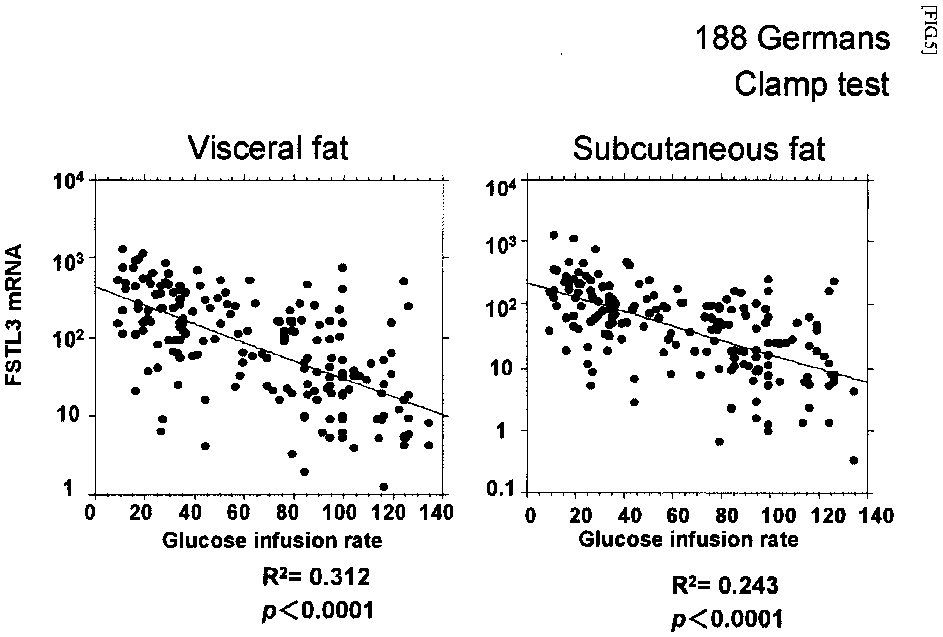

To verify the result of the first example, the inventors examined correlation between expression of FSTL3 mRNA and BMI for 188 cases of German subjects independent of the 113 cases described above. The details of the 188 cases include 108 male and 80 female subjects of an average age of 55.3±15.2, of which 145 were non-diabetic subjects, 5 were IGT subjects, and 38 were type-2 diabetic subjects. In both the visceral fat and subcutaneous fat samples, strong positive correlation was identified between the level of FSTL3 mRNA expression and BMI (see Fig. 3). Similarly, in both the visceral fat and subcutaneous fat samples, strong positive correlation was identified between the level of FSTL3 mRNA expression and abdominal circumference (see Fig. 4). On the other hand, significant negative correlation was identified between the level of FSTL3 mRNA expression and insulin sensitivity (clamp GIR) (see Fig. 5). From Figs. 3 to 5, it is apparent that when the level of FSTL3 mRNA expression is higher, BMI value tends to be higher, that when the level of FSTL3 mRNA expression is higher, abdominal circumference tends to be larger, and that when the level of FSTL3 mRNA expression higher, insulin sensitivity tends to be lower. Therefore, we found that it is possible to infer a higher risk of developing diabetes or obesity if the level of FSTL3 mRNA expression is increased by a certain multiple or more as compared to an established standard value of FSTL3 mRNA expression level. The FSTL3 mRNA expression level is represented as a multiple when an expression level of 18sRNA in the same sample is defined as one.

In addition to BMI, abdominal circumference (waist), and insulin sensitivity (clamp GIR) described above, correlations of body fat, a visceral fat area (V), a subcutaneous fat area (S), HbA1c, HOMA-IR, and HOMA-β with the level of FSTL3 mRNA expression were investigated. Significant correlation was found between the level of FSTL3 mRNA expression and all of these indices. The results are described in Table 4.

**[TABLE 4]**

| Correlation with FSTL3 (188 Germans) | | |
|---|---|---|
| | FSTL3 (visceral fat) | FSTL3 (subcutaneous fat) |
| Waist | *p*<0.0001 | *p*<0.0001 |
| BMI | *p*<0.0001 | *p*<0.0001 |
| Body fat | *p*<0.0001 | *p*<0.0001 |
| Visceral fat area (V) | *p*<0.0001 | *p*<0.0001 |
| Subcutaneous fat area (S) | *p*<0.0001 | *p*<0.0001 |
| Clamp GIR | *p*<0.0001(inverse correlation) | *p*<0.0001(inverse correlation) |
| HbA1c | *p* = 0.01 | *p* = 0.0088 |
| HOMA-IR | *p* = 0.0203 | *p* = 0.018 |
| HOMA-*β* | *p* = 0.0042 | *p* = 0.014 |

Since Patent Literature 1 does not disclose biological samples and case groups used, it is not necessarily clear what factor caused the difference between Patent Literature 1 and this study. However, since "Brief Description of Drawings" in paragraph 0115 of Patent Literature 1 includes a description of "type-2 diabetic patients", it can be determined that samples from patients who had already developed diabetes were used in Patent Literature 1, unlike the cases of from normal to obesity used in this study.

### [EXAMPLE 3]

The inventors performed the Northern blot analysis by using mRNA extracted from kidney, skeletal muscle, pancreas, liver, subcutaneous fat, visceral fat, brain, and testis to examine tissue-specific expression of FSTL3 mRNA. The analysis was performed for *db*/*db* mice (*db*/*db*: BKS.Cg-+ *Lepr^{db}*/+ *Lepr^{db}*/Jcl* *Lepr^{db}*/+ *Lepr^{db}*, eight-week-old in the obesity/diabetes state) that are genetic obesity models and control mice (mysty: BKS.Cg-m +/+ *Lepr^{db}*/Jcl*m).

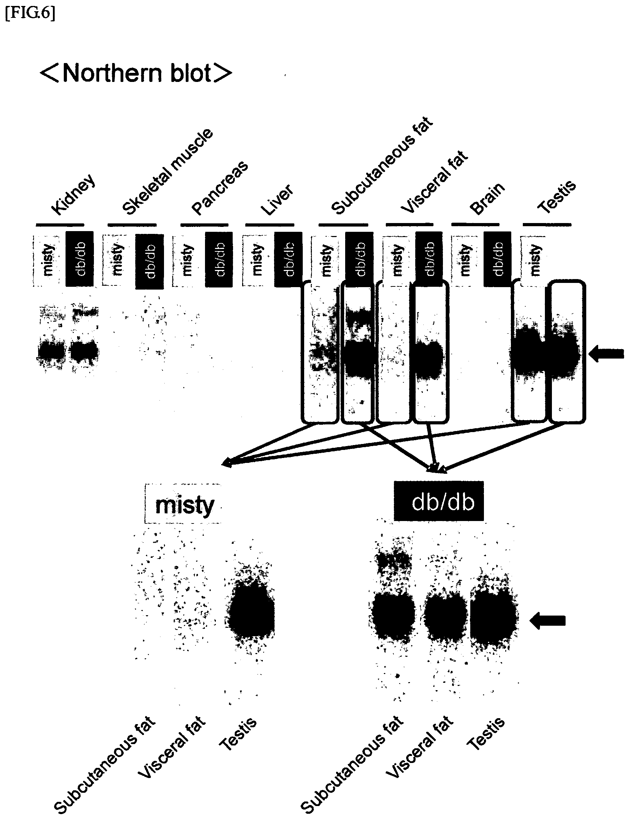

In both control and *db*/*db* mice, the tissue-specific expression of FSTL3 mRNA was identified in the kidney and the testis (see Fig. 6). On the other hand, in the subcutaneous fat and the visceral fat, the tissue-specific expression of FSTL3 mRNA was identified only in *db*/*db* mice. From this result, it was found out that the expression of FSTL3 mRNA in the subcutaneous fat and the visceral fat is raised in obese individuals regardless of the difference between species of human and mouse.

### [EXAMPLE 4]

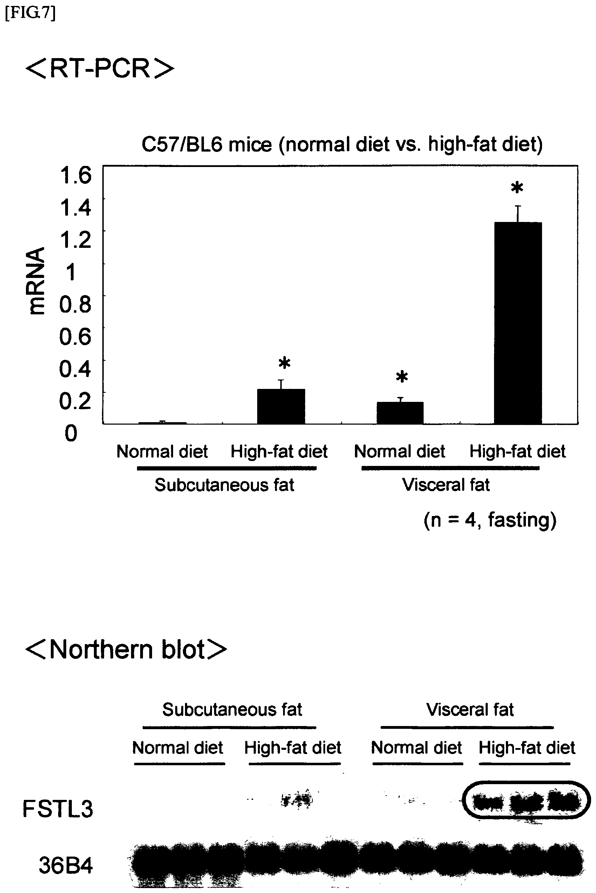

The expression of FSTL3 mRNA in the subcutaneous fat and the visceral fat was examined with the RT-PCR method and the Northern blot analysis when normal diet (Japan Laboratory Animals, Inc., mouse food MF) or high-fat diet (Clea High Fat diet 32, crude fat 32 %, fat-derived calorie 60 %) was fed to normal mice (C57/BL6 mice). Although the mice were put in an obese condition due to the high-fat diet, the collection of subcutaneous and visceral fat samples were performed during fasting. This was intended to eliminate the possibility of a temporary elevation in expression due to feeding. In both detection systems, i.e., the RT-PCR method and the Northern blot analysis, an elevation in the expression of FSTL3 mRNA was observed in the groups eating the high-fat diet. In comparing the subcutaneous fat and the visceral fat, higher expression was detected in the visceral fat (see Fig. 7).

### [EXAMPLE 5]

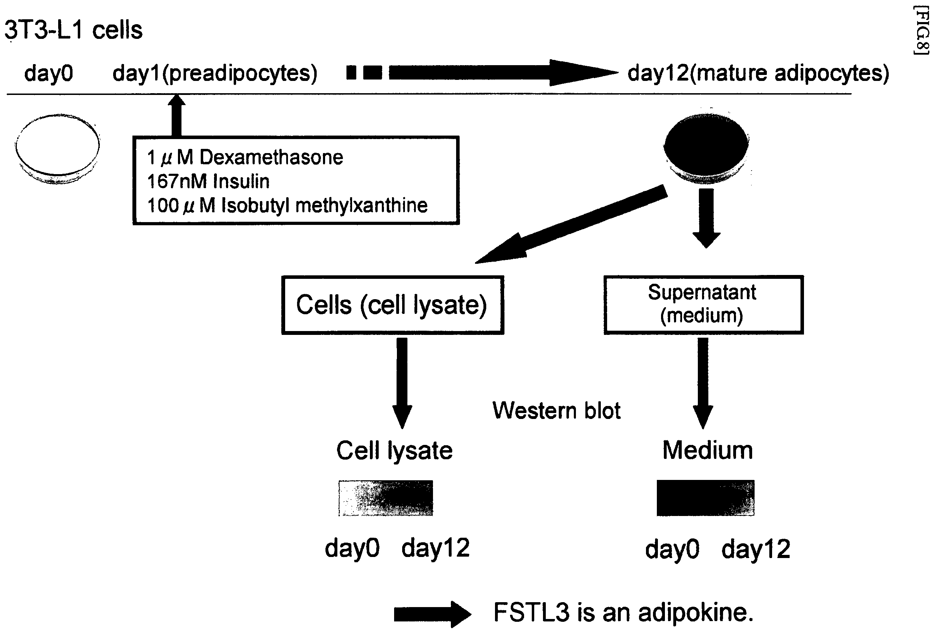

With regard to the 3T3-L1 mouse embryonic fibroblasts, differentiation of preadipocytes to mature adipocytes can be induced. The 3T3-L1 cells were acquired from ATCC. The composition of test medium was DMEM (Gibco 11995), 10 % FBS (GIBCO 10437), and 1 % penicillin/streptomycin (P/S) and the cells were cultured at a CO₂ concentration of 5 % and a temperature of 37.0 °C. After administration to the 3T3-L1 cells (day 1) of 1 µM dexamethasone, 167 nM insulin, and 100 µM isobutylmethylxanthine, the cells were cultured for 11 days and differentiated to mature adipocytes (day 12). The cells and the culture fluid supernatant of the cells were recovered to check whether FSTL3 protein was expressed with Western blotting. While the expression of FSTL3 protein was not found or very little in the 3T3-L1 cells before the induction of differentiation (day 0), it was identified that FSTL3 protein was expressed in both the cells and the culture fluid supernatant after differentiation to mature adipocytes (day 12) (see Fig. 8). From this result, it was found out that FSTL3 is an adipokine secreted extracellularly from mature adipocytes.

The fact that FSTL3 protein is secreted to the extracellular space of adipocytes means that body fluid (e.g., blood, serum) of a subject can be used for examination when FSTL3 protein or a metabolic product thereof is used as a diagnostic marker of obesity. This is a non-invasive method as compared to the collection of subcutaneous fat or visceral fat and is advantageous for use in routine examination.

### [EXAMPLE 6]

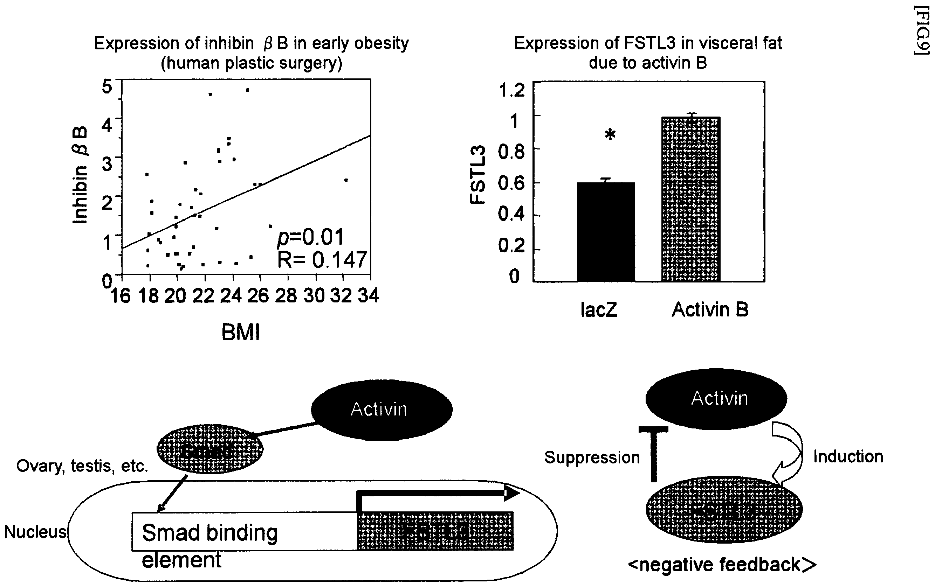

From the results of the first to fifth examples, it was found out that FSTL3 is useful for determination of risk of developing diabetes or obesity. FSTL3 is a protein known that binds to activins to inhibit binding of activins to their receptors. In this regard, the inventors paid attention to the behavior of activins. For the behavior of inhibin βB gene that is one of the genes coding activins, correlation was examined between the level of inhibin βB mRNA expression and BMI value in early obesity. A homodimer formed from the product of inhibin βB gene is referred to as activin B. Significant positive correlation was found between the level of inhibin βB mRNA expression and BMI value in early obesity (see upper left of Fig. 9).

Adenovirus recombined with inhibin βB gene was administered to mice to examine the FSTL3 mRNA expression level in the visceral fat. When activin B was expressed in the visceral fat, a rise in expression of FSTL3 mRNA was observed as compared to control mice (lacZ) (see upper right of Fig. 9).

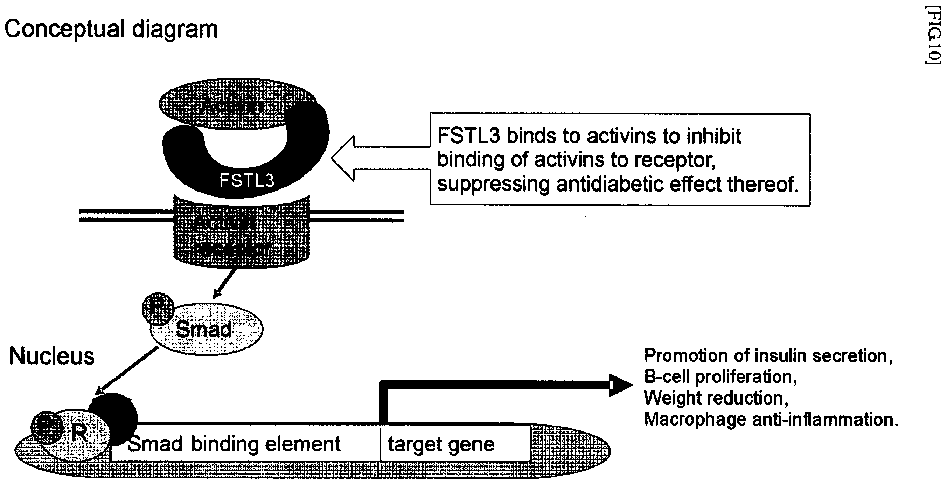

When we consider the molecular mechanism in which FSTL3 suppresses the antidiabetic effect of activin B through associating with activin B, and thus, inhibiting the binding of activin B to the receptor (see Fig. 10; unpublished data of the inventors), the up-regulation of FSTL3 by activin B described above is surprising. By comprehensively considering these data, it is believed that activin B activates Smad through the binding of activin B to the receptor (e.g., ALK7; see Non-Patent Literature 3) to up-regulate the expression of FSTL3. On the other hand, it is believed that FSTL3 associates with activin B to inhibit the binding of activin B to the receptor, suppressing the effect of activin B.

Therefore, it is believed that in a normal physiological state, a negative feedback mechanism works between activin B and FSTL3 to keep balance therebetween. When we consider the competing actions of activin B and FSTL3, use of the ratio of expression levels of activin B and FSTL3 as an index of obesity degree is an effective approach.

### [EXAMPLE 7]

For 188 cases of German subjects, the ratio of expression level of FSTL3 mRNA to inhibin βB mRNA was obtained to examine the relationship with BMI value.

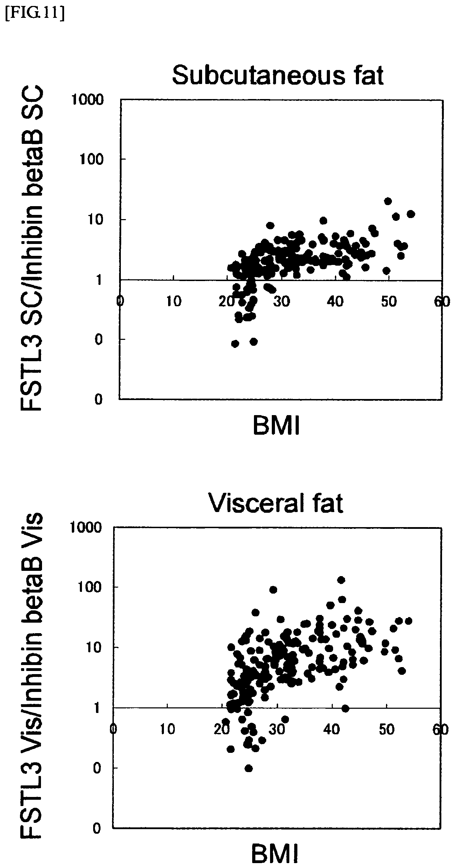

As a result, it was confirmed that individuals with a BMI value of 25 or greater extremely frequently had a ratio of (FSTL3 mRNA expression level)/(inhibin βB mRNA expression level) greater than one (see Fig. 11). Therefore, it was found out that the ratio of expression level of FSTL3 gene to inhibin βB gene in a biological sample can be correlated with the detection of a degree of risk of developing obesity or diabetes. The level of FSTL3 mRNA expression is represented by a multiple where the level of 18sRNA expression in the same sample is defined as one and the level of inhibin βB mRNA expression is represented by a multiple where the level of cyclophilin A expression in the same sample is defined as one.

### [EXAMPLE 8]

The C57BL/6J mice were used for comparing blood glucose levels between mice with FSTL3 gene expressed by adenovirus in the liver and control mice (lacZ) until 120 minutes had elapsed in a glucose load test.

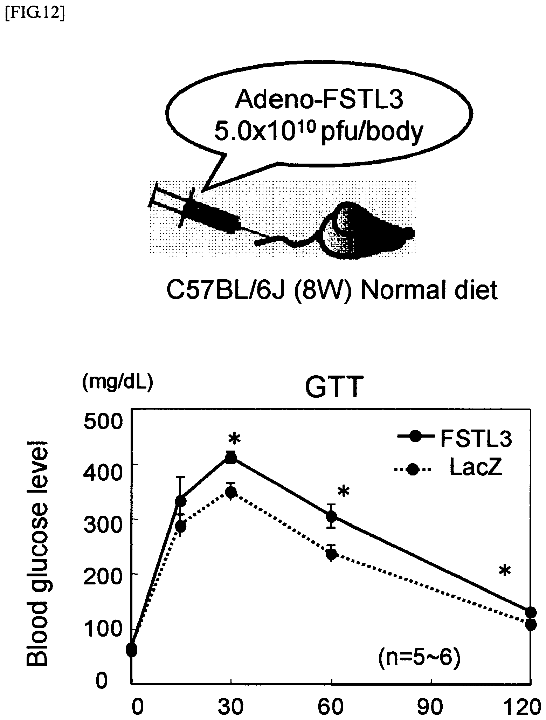

As a result, it was recognized that the glucose tolerance is deteriorated by expressing FSTL3 gene in the liver (see Fig. 12).

### [EXAMPLE 9]

The *db*/*db* mice, an obesity/diabetes model, were used for comparing blood glucose levels between *db*/*db* mice administered with FSTL3 antisense oligonucleotides and control *db*/*db* mice without administration until 120 minutes had elapsed in a glucose load test.

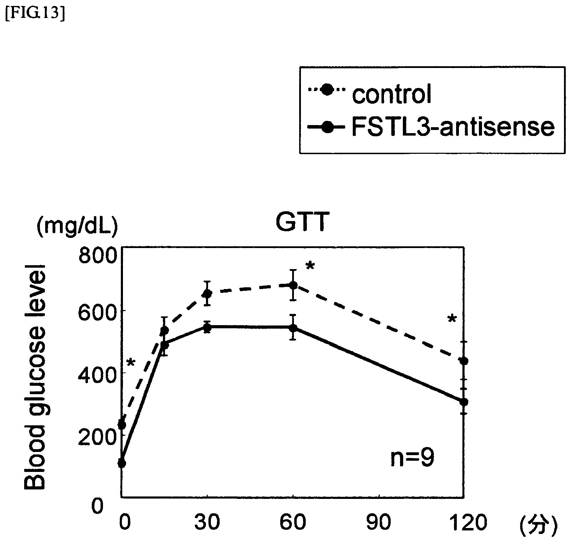

As a result, improvement in glucose tolerance is recognized in the mice administered with antisense oligonucleotides as compared to the control mice (see Fig. 13).

### [EXAMPLE 10]

Akt phosphorylation signals in the liver and the skeletal muscle were measured in the *db*/*db* mice administered with FSTL3 antisense oligonucleotides and the control *db*/*db* mice. The mice used in the ninth example were dissected after 31 days from the first day of the administration to extract the livers and the skeletal muscles and the Akt phosphorylation signals were measured in each of the regions.

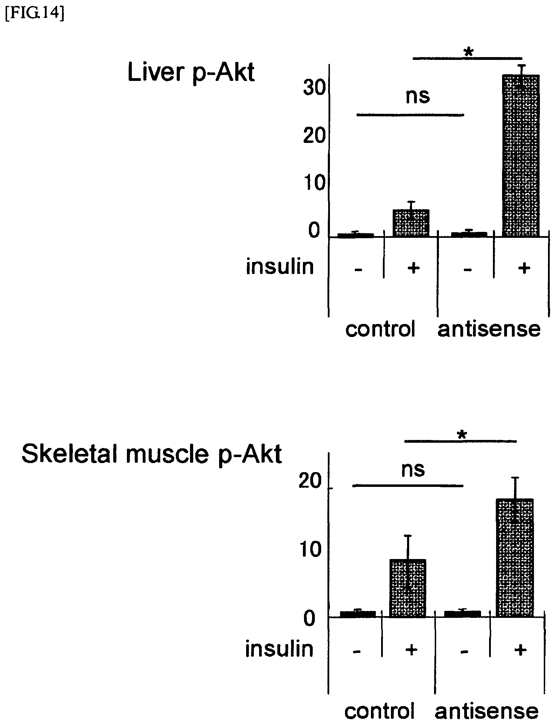

As a result, the phosphorylation of Akt was identified in both the livers and the skeletal muscles in the mice administered with FSTL3 antisense oligonucleotides (see Fig. 14). This indicated that FSTL3 is involved in induction of insulin resistance through inhibition of insulin signaling via Akt and it was found out that FSTL3 is an index (marker) suitable for detecting a risk of developing obesity and/or diabetes in early phase.

### [EXAMPLE 11]

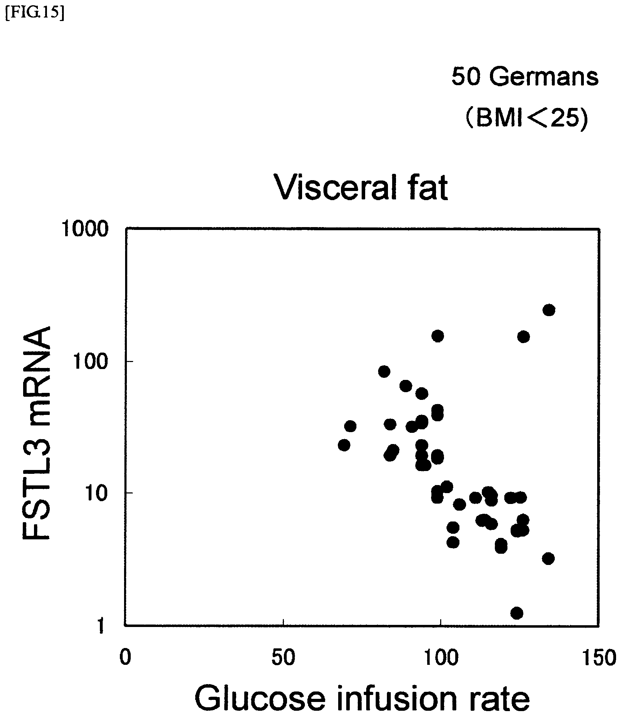

For 50 cases with a BMI value less than 25 out of the 188 cases of the second example, the relationship between insulin sensitivity (glucose infusion rate in clamp test) and FSTL3 mRNA expression level in the visceral fat was analyzed. The relationship between FSTL3 mRNA expression level and glucose infusion rate is as depicted in Fig. 15.

The Spearman rank-correlation coefficient is -0.6175, P<0.0001 and, therefore, Significant negative correlation was confirmed. Therefore, it is believed that using an FSTL3 mRNA expression level in an individual with a BMI value less than 25 (normal value range in terms of obesity) as an index of preliminary stage of diabetes or preliminary stage of obesity is an effective approach.

## Claims

1. A method of detecting a risk of developing obesity, comprising
(i) a step of measuring an expression level of FSTL3 gene in a biological sample selected from the group consisting of blood sample, serum sample, plasma sample, visceral fat tissue sample and subcutaneous fat tissue sample derived from an individual having a BMI value less than 25, wherein
if the expression level of FSTL3 gene in the biological sample is increased by a certain multiple or more as compared to a standard value S_{FSTL3}, it is determined that the risk of developing obesity is high.

2. The method according to claim 1, further comprising
(ii) a step of measuring an expression level of inhibin βB gene in the biological sample, wherein
if the expression level of FSTL3 gene in the biological sample is increased by a certain multiple or more as compared to a standard value S_{FSTL3} and the ratio of expression level of FSTL3 gene to inhibin βB gene in the biological sample is equal to or greater than a certain multiple of a standard value S_{FSTL3/ActB}, it is determined that the risk of developing obesity is high.

## Patentansprüche

1. Verfahren zum Detektieren eines Risikos, Fettleibigkeit zu entwickeln, umfassend
(i) einen Schritt des Messens eines FSTL3-Gen-Expressionsniveaus in einer biologischen Probe, ausgewählt aus der Gruppe bestehend aus Blutprobe, Serumprobe, Plasmaprobe, Probe von viszeralem Fettgewebe und Probe von subkutanem Fettgewebe, abgeleitet von einem Individuum mit einem BMI-Wert, der geringer als 25 ist, wobei
wenn das FSTL3-Gen-Expressionsniveau in der biologischen Probe um ein bestimmtes Vielfaches oder mehr verglichen mit einem Standardwert S_{FSTL3} erhöht ist, festgestellt wird, dass das Risiko, Fettleibigkeit zu entwickeln, hoch ist.

2. Verfahren gemäß Anspruch 1, weiter umfassend
(ii) einen Schritt des Messens eines Inhibin-βB-Gen-Expressionsniveaus in der biologischen Probe, wobei
wenn das FSTL3-Gen-Expressionsniveau in der biologischen Probe um ein bestimmtes Vielfaches oder mehr verglichen mit einem Standardwert S_{FSTL3} erhöht ist und das Verhältnis des Expressionsniveaus vom FSTL3-Gen zum Inhibin-βB-Gen in der biologischen Probe gleich zu oder größer als ein bestimmtes Vielfaches eines Standardwerts S_{FSTL3/ActB} ist, festgestellt wird, dass das Risiko, Fettleibigkeit zu entwickeln, hoch ist.

## Revendications

1. Procédé de détection d'un risque de développement de l'obésité, comprenant :
(i) une étape de mesure d'un niveau d'expression du gène FSTL3 dans un échantillon biologique choisi parmi le groupe comprenant un échantillon de sang, un échantillon de sérum, un échantillon de plasma, un échantillon de tissu graisseux viscéral et un échantillon de tissu graisseux sous-cutané provenant d'un individu présentant une valeur d'IMC inférieure à 25, dans lequel
si le niveau d'expression du gène FSTL3 dans l'échantillon biologique est augmenté par un certain multiple ou plus par comparaison à la valeur standard S_{FSTL3}, il est déterminé que le risque de développement de l'obésité est élevé.

2. Procédé selon la revendication 1, comprenant en outre :
(ii) une étape de mesure d'un niveau d'expression du gène d'inhibine βB dans l'échantillon biologique, dans lequel
si le niveau d'expression du gène FSTL3 dans l'échantillon biologique est augmenté par un certain multiple ou plus par comparaison à la valeur standard S_{FSTL3} et le rapport de niveau d'expression du gène FSTL3 par rapport au gène d'inhibine βB dans l'échantillon biologique est supérieur ou égal à un certain multiple d'une valeur standard S_{FSTL3/ActB}, il est déterminé que le risque de développement de l'obésité est élevé.


### SEQUENCE LISTING

<110> The University of Tokyo SEKISUI MEDICAL CO., LTD
<120> Method for detecting the risk of obesity and diabetes
<130> 10P00544
<140> EP 11786754.9
   <141> 2011-05-27
<150> JP 2010-122148
   <151> 2010-05-27
<160> 3
<170> PatentIn version 3.1
<210> 1
   <211> 771
   <212> DNA
   <213> Mus musculus
<400> 1
   atgcgttctg gggcactgtg gccgctgctt tggggagccc tggtctggac agtgggatcc      60
   gtgggcgccg tgatgggctc cgaggattct gtgcccggtg gcgtgtgctg gctccagcag     120
   ggcagagagg ccacctgcag tctggtgctg aagactcgtg tcagccggga ggagtgctgt     180
   gcttccggca acatcaacac cgcctggtcc aacttcaccc acccaggcaa taaaatcagc     240
   ctgctagggt tcctgggcct cgtccactgc ctcccctgca aagattcctg cgacggagtg     300
   gagtgcggcc ccggcaaggc gtgccgcatg ctggggggcc gtccacactg cgagtgcgtg     360
   cccaactgcg aggggcttcc cgcgggcttc caggtctgcg gctctgatgg cgccacctac     420
   cgggacgaat gcgaactgcg caccgcgcgc tgtcgcggac acccagactt gcgcgtcatg     480
   taccgcggcc gctgtcaaaa gtcttgcgct caggtagtgt gcccgcgtcc ccagtcgtgc     540
   cttgtggatc agaccggcag cgcacactgc gtggtgtgtc gcgctgcgcc ctgcccagta     600
   ccttccaacc ccggccaaga actctgtggc aacaacaacg ttacctacat ctcgtcgtgt     660
   cacctgcgcc aggccacttg cttcctgggc cgctccattg gggttcggca cccaggcatc     720
   tgcacaggtg gccccaaagt accagcagag gaggaagaga acttcgtgtg a              771
<210> 2
   <211> 24
   <212> DNA
   <213> Artificial Sequence
<220>
   <223> antisense oligo
<400> 2
   ggguuggaag gtactgggca gggc 24
<210> 3
   <211> 24
   <212> DNA
   <213> Artificial Sequence
<220>
   <223> control oligo
<400> 3
   cgggacgggt catggaaggu uggg 24